# FASCICULE DE BREVET EUROPEEN

(11) **EP 1 656 075 B1**
(45) Date de publication et mention de la délivrance du brevet: **07.10.2009**
(21) Numéro de dépôt: 04786326.1
(22) Date de dépôt: 19.08.2004
(51) Int. Cl.: A61B 17/70

(54) **IMPLANT INTERVERTEBRAL POUR L`ARTICULATION LOMBO-SACREE**
INTERVERTEBRAL-IMPLANTAT FÜR DAS LUMBO-SAKRAL-GELENK
INTERVERTEBRAL IMPLANT FOR THE LUMBOSACRAL JOINT

(30) Priorité: 21.08.2003 FR 0310063
(43) Date de publication de la demande: 17.05.2006
(73) Titulaire: ZIMMER SPINE, 33000 Bordeaux (FR)
(72) Inventeur: PASQUET, Denis, 13100 Aix en Provence (FR); LE COUEDIC, Régis, F-78570 ANDRESY (FR); SENEGAS, Jacques, F-33700 MERIGNAC (FR); RENAUD, Christian, F-81160 ARTHES (FR)
(74) Mandataire: Besnard, Christophe Laurent
(86) Numéro de dépôt international: PCT/FR2004/002160
(87) Numéro de publication internationale: WO 2005/020860

(56) Documents cités:
- EP-A- 1 138 268
- FR-A- 2 714 591
- FR-A- 2 799 640
- FR-A- 2 822 051
- US-A- 5 810 815

## Description

La présente invention a pour objet un implant intervertébral pour l'articulation lombo-sacrée.

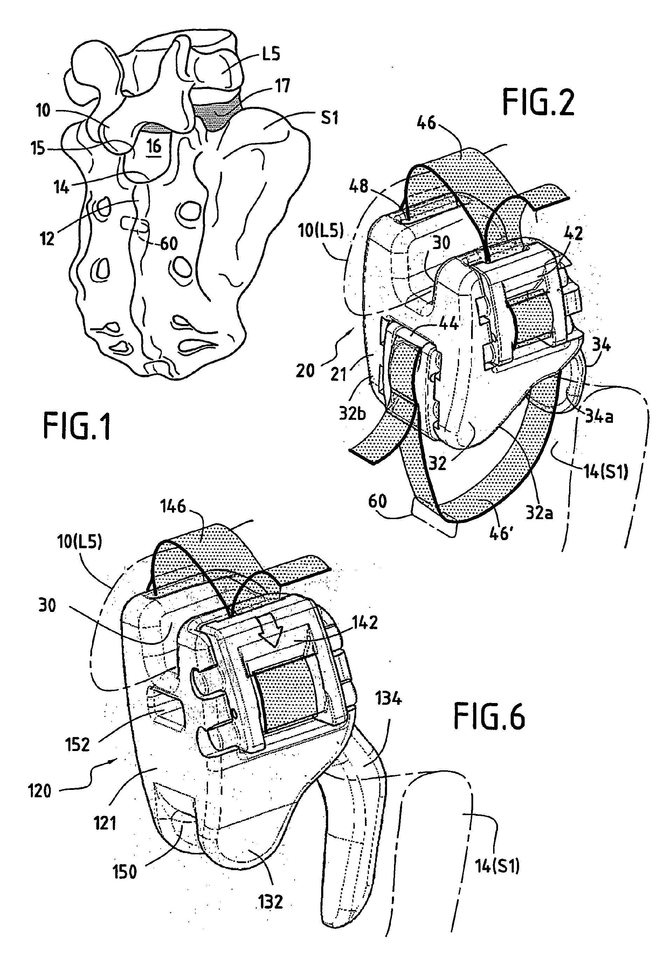

Dans l'anatomie du rachis, le sacrum, situé en dessous des vertèbres lombaires, est constitué par cinq vertèbres qui au fil de l'évolution humaine se sont soudées entre elles. La vertèbre supérieure du sacrum, notée S1, est articulée à la cinquième vertèbre lombaire, notée L5. Cette articulation constitue l'articulation lombo-sacrée, ou articulation L5-S1, représentée figure 1.

Les vertèbres lombaires présentent une saillie médiane et postérieure : l'apophyse épineuse, dénommée ci-après épineuse 10. Les vertèbres sacrées, elles, ont perdu leurs épineuses au fil de l'évolution, et ne conservent à la place qu'une petite bosse résiduelle 12.

Chez l'homme, certaines douleurs dorsales peuvent être dues aux contraintes, liées au déplacement relatif de deux vertèbres entre elles, qui s'exercent sur le disque intervertébral situé entre ces vertèbres.

On connaît déjà de nombreux implants intervertébraux qui visent à limiter le déplacement de deux vertèbres entre elles de manière à soulager le disque intervertébral, et notamment celui décrit dans le document FR 2 775 183. Cet implant est une cale présentant sur ces faces supérieure et inférieure deux évidements longitudinaux orientés dans la même direction, celle du plan médian de la cale, et destinés à recevoir les épineuses des vertèbres adjacentes entre lesquelles la cale sera implantée. La cale est ensuite maintenue en position par des sangles entourant lesdites épineuses. En bloquant une partie du rachis, la cale produit un transfert de charge au-dessus et en-dessous des vertèbres concernées et soulage ainsi le disque intervertébral situé entre ces vertèbres. Or, du fait de l'anatomie de la région sacrée, et plus particulièrement de l'absence d'épineuse sur la vertèbre S1, la mise en place de ce type de cale est impossible au niveau de l'articulation L5-S1.

On connaît également un type d'implant décrit dans le document EP 1 138 268, adapté cette fois à l'anatomie de la région lombo-sacrée. Cet implant comprend une cale intervertébrale et une barre de liaison. La cale intervertébrale présente deux évidements sensiblement orthogonaux entre eux, et la barre de liaison est rendue solidaire du sacrum au moyen de deux crochets fixés à la vertèbre S1. Plus précisément, ces crochets prennent appui sur la partie supérieure de la vertèbre S1, appelée également arc postérieur, et sont fixés sur le sacrum chacun par des moyens de fixation, comme des agrafes, qui permettent de positionner et de stabiliser les crochets. Une fois les crochets installés sur le sacrum, la barre est fixée aux crochets, et la cale intervertébrale est mise en place. L'évidement supérieur de la cale est apte à recevoir l'épineuse de la vertèbre L5 et l'évidemment inférieur présente une forme appropriée pour recevoir la barre, de sorte que la cale repose sur cette barre.

Ce type d'implant présente néanmoins plusieurs inconvénients. D'abord, à court terme, la fixation des crochets sur le sacrum, par agrafage par exemple, peut traumatiser le patient. Ensuite, à moyen et long terme, les contraintes en compression et en extension auxquelles l'implant est soumis vont se répercuter sur les moyens de fixation des crochets au sacrum, et entraîner l'élargissement des trous dans lesquels ces moyens de fixation sont logés. Un jeu va alors se créer entre le sacrum et les crochets, et conduire à un mauvais comportement mécanique de l'implant, voire à l'arrachement des moyens de fixation. Le traumatisme subi par le patient est alors important, et une nouvelle opération doit être envisagée pour retirer et éventuellement remplacer l'implant défectueux.

La présente invention se propose de résoudre les inconvénients des dispositifs existants.

Dans ce but, l'invention a pour objet un implant intervertébral pour l'articulation lombo-sacrée, qui consiste en une cale apte à être disposée entre une cinquième vertèbre lombaire L5 et la vertèbre S1 du sacrum articulée à cette dernière. Le corps de ladite cale présente deux faces d'extrémité supérieure et inférieure opposées. La cale présente une rainure, orientée suivant le plan médian de la cale, ménagée sur la face d'extrémité supérieure et apte à recevoir l'épineuse de ladite vertèbre lombaire L5. La cale présente également un logement longitudinal, orienté orthogonalement à ladite rainure, ménagé sur la face d'extrémité inférieure et apte à recevoir la partie supérieure de la vertèbre S1 du sacrum, de sorte que la cale repose directement sur cette partie supérieure.

L'implant se réduit ainsi à une cale intervertébrale, et cette cale peut être mise en contact direct avec la vertèbre sacrée S1 sans qu'il soit nécessaire d'utiliser d'autres éléments comme une barre de fixation, ce qui facilite sa mise en place.

Selon l'invention, le corps de ladite cale présente des première et deuxième faces latérales opposées dans lesquelles ladite rainure débouche, et présente à son extrémité inférieure une extension dont un premier côté est dans le prolongement de la première face latérale, et dont un deuxième côté, opposé au premier, définit un renfoncement par rapport à la deuxième face latérale du corps de la cale, la cale comprenant également une patte, de largeur inférieure à la largeur du corps de la cale selon la direction orthogonale au plan médian de la cale, raccordée au corps de la cale, et qui s'étend en regard du deuxième côté de ladite extension, de sorte que la face interne de ladite patte, orientée face au deuxième côté de l'extension, définit avec ce deuxième côté le contour dudit logement.

La largeur réduite de la patte permet de mieux adapter la forme de l'implant à l'anatomie de la région sacrée. En effet, la partie supérieure de la vertèbre S1 du sacrum forme un arc postérieur. Cet arc postérieur est concave et définit avec la partie antérieure du sacrum, appelée corps vertébral, un orifice traversé par la moelle épinière : le trou rachidien. L'invention vise à limiter l'espace occupé par la patte à l'intérieur de cet orifice pour laisser le plus d'espace possible à la moelle épinière, et éviter ainsi de soumettre cette dernière à des contraintes qui se traduisent généralement par des douleurs chez le patient.

Dans ce but, la largeur de la patte est suffisamment réduite par rapport à celle corps de la cale pour s'engager profondément dans la cavité formée par l'arc postérieur. Les dimensions de la patte ne peuvent toutefois pas être réduites au point de risquer sa rupture sous l'effet des contraintes auxquelles elle est soumise. La largeur retenue doit donc tenir compte des propriétés mécaniques du matériau utilisé pour réaliser ladite patte.

On notera que la surface du corps de la cale située au niveau du fond du logement, c'est à dire à la base de la patte, constitue la surface d'appui de la cale sur le bord supérieur de l'arc postérieur du sacrum, et que le corps de la cale doit donc à ce niveau avoir une largeur suffisante pour assurer un appui stable.

Avantageusement, la face intérieure de ladite patte située en regard du renfoncement peut être convexe de manière à présenter une forme complémentaire de celle de la face interne de l'arc postérieur, tournée vers le corps vertébral. Cette caractéristique permet à la patte d'épouser la forme de cette paroi et donc d'occuper un espace limité à l'intérieur du trou rachidien.

Avantageusement, la section, suivant le plan médian de la cale, du logement ménagé sur la face d'extrémité inférieure et apte à recevoir la partie supérieure de la vertèbre S1, a la forme générale d'un U, et le plan médian de ce logement n'est pas orthogonal au plan moyen défini par le fond de ladite rainure.

La forme et l'inclinaison particulière du logement visent à améliorer l'appui de la cale sur l'arc postérieur de la vertèbre S1, qui n'est pas orthogonal à la partie de l'épineuse de la vertèbre L5 destinée à être au contact du fond de la rainure, et dont le bord supérieur présente une forme convexe. Ces caractéristiques permettent également de faciliter la mise en place de la cale.

Avantageusement, le plan médian dudit logement est incliné par rapport au plan moyen défini par le fond de ladite rainure d'un angle compris entre 40 et 80°. Cette inclinaison garantit la stabilité de la cale lorsqu'elle est en place.

Le contour du logement étant défini par la face interne de la patte et le deuxième côté de ladite extension, l'orientation du plan médian du logement par rapport au plan moyen défini par le fond de la rainure, dépend de l'inclinaison générale de la face interne de la patte et du deuxième côté de l'extension.

Ainsi, avantageusement, une zone de la face interne de la patte est inclinée par rapport au plan moyen défini par le fond de ladite rainure d'un angle A compris entre 60 et 80°, de préférence sensiblement égal à 70°, et une partie du deuxième côté de l'extension est inclinée par rapport au plan moyen défini par le fond de ladite rainure d'un angle B compris entre 40 et 70°, de préférence entre 50 et 60°.

Selon un autre mode de réalisation particulier de l'invention, une échancrure est ménagée dans ladite extension, en face de ladite patte. La bosse située sur la face postérieure du sacrum, trace résiduelle de la présence d'épineuse sur la vertèbre sacrée S1, va ainsi venir se loger à l'intérieur de cette échancrure lors de la mise en place de l'implant, ce qui permet d'améliorer la stabilité de ce dernier.

L'invention et ses avantages seront mieux compris à la lecture de la description détaillée de deux modes de réalisation de l'invention représentés sur les figures suivantes.
- La figure 1 représente schématiquement l'anatomie de la région lombo-sacrée du rachis ;
- la figure 2 représente un premier mode de réalisation de l'implant de l'invention, mis en place entre la vertèbre lombaire L5 et la vertèbre du sacrum S1 ;
- les figures 3 et 4 représentent lesdites première et deuxième faces latérales du corps de la cale de la figure 2 ;
- la figure 5 est une vue en coupe du corps de la cale de la figure 2 selon son plan médian M ;
- la figure 6 représente un second mode de réalisation de l'implant de l'invention, mis en place entre la vertèbre lombaire L5 et la vertèbre du sacrum S1 ;
- les figures 7 et 8 représentent lesdites première et deuxième faces latérales du corps de la cale de la figure 6 ;
- la figure 9 est une vue en coupe du corps de la cale de la figure 6 selon son plan médian M'.

La cinquième vertèbre lombaire L5 et la vertèbre supérieure du sacrum S1 sont représentées schématiquement figure 1. La vertèbre L5 présente dans sa partie médiane et postérieure une épineuse 10. Cette épineuse 10 est située dans le plan sagittal du rachis. La vertèbre S1, elle, ne possède pas d'épineuse, mais présente à la place sur sa face postérieure une bosse résiduelle 12.

La partie supérieure de la vertèbre S1 forme un arc postérieur 14. La face interne de l'arc postérieur fait face au corps vertébral 15 du sacrum, elle est concave et définit avec le corps vertébral un orifice traversé par la moelle épinière (non représentée), appelé trou rachidien 16.

L'implant de l'invention comme représenté figures 2 et 6 est apte à être mis en place entre l'épineuse 10 et l'arc postérieur 14. Il permet de limiter le mouvement de la vertèbre L5 par rapport à la vertèbre S1 et permet ainsi de soulager le disque intervertébral 17 situé entre ces deux vertèbres, des contraintes qui résultent dudit mouvement.

En référence aux figures 2 à 5, on va maintenant décrire un premier mode de réalisation d'implant intervertébral selon l'invention.

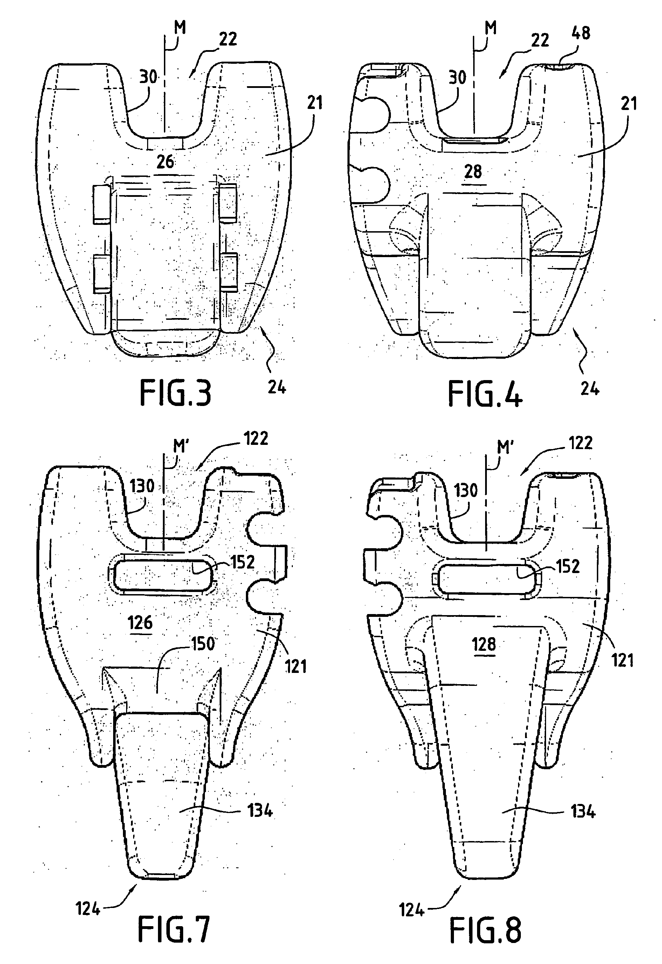

Cet implant consiste en une cale 20 dont le corps 21 est représenté figures 3 à 5. Le corps de la cale est réalisé en matériau biocompatible, par exemple un bio-polymère. Dans le mode de réalisation choisi, le corps 21 est moulé en polyétheréthercétone, noté ci après PEEK. Ce polymère est du type de celui commercialisé notamment sous la marque PEEK®.

Le corps 21 de la cale présente une face d'extrémité supérieure 22 et une face d'extrémité inférieure 24 opposée à la face 22. Il présente également une première et une deuxième face latérale opposées 26 et 28.

Le plan médian M de la cale est sécant aux faces 22, 24, 26 et 28, et sépare le corps 21 en deux parties quasi-symétriques, qui ne diffèrent entre elles que par la présence sur l'une des parties d'une cavité susceptible de recevoir des moyens de fixation. Lorsque la cale est mise en place sur le rachis, le plan M correspond sensiblement au plan sagittal du rachis.

Une rainure 30 orientée suivant le plan médian M de la cale 20 est ménagée sur la face d'extrémité supérieure 22 du corps 21, et est apte à recevoir l'épineuse 10 de la vertèbre lombaire L5. Cette rainure débouche dans les faces latérales 26 et 28. La section de la rainure 30 selon le plan perpendiculaire au plan médian M de la cale, a sensiblement la forme d'un U à base sensiblement plane. Cette forme lui permet de recevoir la partie inférieure de l'épineuse 10.

Le corps 21 de la cale 20 présente à son extrémité inférieure une extension 32 dont un premier côté 32b est dans le prolongement de la première face latérale 26, et dont un deuxième côté 32a opposé au premier, définit un renfoncement par rapport à la deuxième face latérale 28 du corps 21 de la cale. La cale 20 comprend en outre une patte 34 raccordée au corps 21 de la cale, et dans le cas présent moulée en une seule pièce avec ce dernier. La patte 34 s'étend en regard du deuxième côté 32a de l'extension 32 et est recourbée : elle s'écarte d'abord du côté 32a en se déportant de la face 26, avant de se rapprocher du côté 32a.

La dimension de la patte 34 suivant la direction orthogonale au plan médian M de la cale 20, ou largeur de la patte, est inférieure à celle du corps 21 de la cale. Par exemple, la largeur de la patte 34 est sensiblement égale à 9mm, alors que la largeur du corps 21, plus particulièrement au voisinage de la patte 34, dans la zone destinée à être en appui sur l'arc postérieur 14 du sacrum, est sensiblement égale à 18 mm, soit le double de la largeur de la patte. L'épaisseur moyenne de la patte 34 est quant à elle sensiblement égale à 2 mm. Pour une patte 34 réalisée en PEEK, ces dimensions sont suffisantes pour que la patte 34 résiste au contraintes auxquelles elle est soumise.

La face interne 34a de la patte 34, orientée face au deuxième côté 32a de l'extension, définit avec ce deuxième côté le contour d'un logement longitudinal 36. Ce logement 36, ménagé sur la face d'extrémité inférieure 24, est orienté orthogonalement à la rainure 30, et est apte à recevoir l'arc postérieur 14, de la vertèbre S1 du sacrum. Ainsi, une fois mise en place, la cale 20 repose directement sur le sacrum.

Pour faciliter le contact entre la cale 20 et l'arc postérieur 14 du sacrum, la section du logement 36 suivant le plan médian M de la cale, a la forme générale d'un U, et le plan médian L du logement 36 n'est pas orthogonal au plan moyen F défini par le fond de la rainure 30.

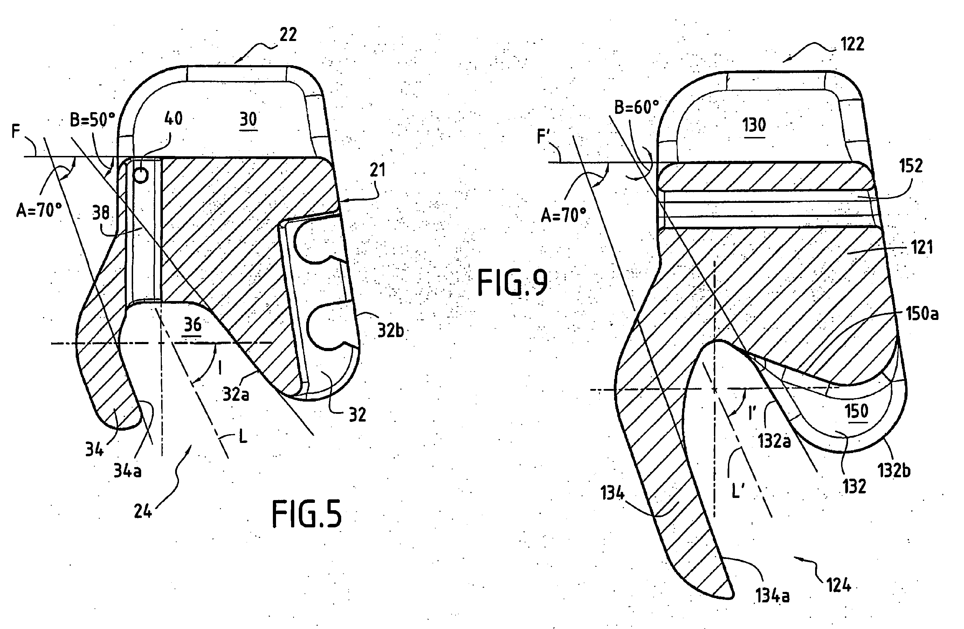

Plus précisément, dans l'exemple représenté, le plan médian L du logement 36 est incliné par rapport au plan moyen F d'un angle I compris entre 50 et 70°. Cette inclinaison dépend d'une part de l'inclinaison générale de la face interne 34a de la patte 34, par rapport au plan F, et d'autre part de l'inclinaison du deuxième côté 32a de l'extension 32 par rapport à ce même plan. Ainsi, une zone de la face interne 34a de la patte 34 est inclinée par rapport au plan moyen F d'un angle A sensiblement égal à 70° comme représenté figure 5, et une partie du deuxième côté 32a de l'extension 32 est inclinée par rapport au plan moyen F d'un angle B sensiblement égal à 50°.

Comme représenté figure 2, la cale comprend en outre des premiers et deuxièmes moyens de fixation 42 et 44 qui vont permettre de fixer le corps 21 de la cale respectivement à l'épineuse 10 de la vertèbre lombaire L5, et à la vertèbre S1 du sacrum.

Ces moyens de fixation sont semblables à ceux décrits dans le document FR-A-2 822 051 et comprennent chacun une sangle 46, 46' et un système d'attache solidaires du corps de la cale.

Le système d'attache est formé par une plaque apte à être logée à l'intérieur d'une cavité de forme complémentaire ménagée dans le corps 21 de la cale. La plaque présente sur deux de ses bords latéraux opposés, des tétons susceptibles d'être engagés à force dans des logements prévus à cet effet dans la cavité du corps 21, de manière à être retenus à l'intérieur de ces derniers. Ceci permet de rendre la plaque solidaire du corps 21 de la cale.

Chaque plaque présente en outre deux fentes à l'intérieur desquelles la sangle 46, 46' est susceptible d'être passée. Ces fentes sont inclinées de manière à autoriser le mouvement de la sangle dans une unique direction correspondant au serrage.

En ce qui concerne les premiers moyens de fixation 42, à sa première extrémité, la sangle 46 est passée dans une ouverture oblongue 48 située le long d'un premier côté de la rainure 30 et traversant le corps 21 de la cale, puis est repliée et cousue sur elle-même de manière à former une boucle. La première extrémité de la sangle 46 est ainsi solidaire du corps de la cale 21.

L'autre extrémité de la sangle 46 est passée dans un système d'attache situé le long du côté de la rainure 30 opposé audit premier côté. Lors de la mise en place de la cale, la sangle 46 est serrée autour de l'épineuse 10.

Les deuxièmes moyens de fixation 44, différents des premiers, sont représentés figures 2 et 5. Comme on le voit sur la figure 5, un trou 38 est ménagé dans le corps 21 de la cale et débouche d'un côté dans le fond du logement 36, au voisinage de la patte 34, et de l'autre côté dans le fond de la rainure 30. Ce trou est susceptible de recevoir une portion de sangle 46'. Au voisinage de la rainure 30, un autre trou 40, est ménagé dans le corps 21. Ce trou 40 croise le trou 38 orthogonalement et est de diamètre inférieur au trou 38. Le trou 40 débouche sur un côté du corps de la cale et est susceptible de recevoir une tige (non représentée).

La sangle 46' est glissée dans le trou 38, et la tige est passée dans une boucle faite à la première extrémité de cette sangle 46'. Ainsi, la première extrémité de la sangle 46' est solidaire du corps 21 de la cale par l'intermédiaire de la tige. L'autre extrémité de la sangle 46' est passée dans le système d'attache 44, situé sur la première face latérale 26 du corps de la cale.

Comme représenté figure 2, lorsque la cale est en place, la sangle 46' passe entre l'arc postérieur 14 du sacrum et la face interne 34a de la patte 34, puis s'étend le long d'une partie de la vertèbre S1, avant de traverser une ouverture 60 pratiquée à cet effet dans le sacrum, et de remonter vers le système d'attache 44.

Dans le cas où le corps 21 de la cale et la patte 34 sont réalisés en PEEK, et la sangle 46' en fil de polyester tissé, la face externe de la patte est plus lisse que celle de la sangle. Il est alors intéressant, car moins traumatisant pour la moelle épinière, de favoriser le .contact entre la patte 34 et la moelle épinière, et de limiter le contact entre la sangle 46' et la moelle épinière. Pour cette raison, la patte 34 recouvre la sangle 46' lorsque la cale est en place.

Un deuxième mode de réalisation de l'implant de l'invention est représenté figures 6 à 9. Cet implant consiste également en une cale 120. La conformation de cette cale étant proche de celle du premier mode de réalisation, les références numériques désignant des parties de la cale 120 semblables à celles de la cale 20 correspondent aux références numériques de la cale 20 augmentées de 100.

La partie supérieure du corps 121 de la cale 120 est identique à celle du corps 21 de la cale 20 ; le corps 121 présente une rainure 130 destinée à recevoir l'épineuse de la vertèbre L5, et des premiers moyens de fixation 142 comprenant une sangle 146 maintenue par un système d'attache et destinée à être serrée autour de l'épineuse 10 de manière à retenir cette dernière à l'intérieur de la rainure 130.

La partie inférieure du corps 121 est différente de celle du corps 21. Tout d'abord la patte 134 est plus longue et plus large que la patte 34. La patte 134, vise à remplacer les moyens de fixation 44 de la cale 20 au sacrum, et doit donc être suffisamment longue pour descendre le long de l'arc postérieur 14 de la vertèbre S1, et assurer le maintien de la cale 120 sur cet arc 14. Par ailleurs, étant soumise à des contraintes importantes, sa largeur et son épaisseur, principalement au voisinage du corps 121 de la cale, doivent être suffisantes pour éviter toute rupture. L'épaisseur de la patte 134 est compensée par l'absence de sangle, de sorte qu'à l'image du premier mode de réalisation, une fois la cale 120 mise en place, la patte 134 occupe le moins d'espace possible à l'intérieur du trou rachidien, de manière à limiter les contacts avec la moelle épinière.

Le logement 136 situé sur la face d'extrémité inférieure 124 de la cale, est défini par la patte 134 et l'extension 132. La section de ce logement 136 suivant l'axe médian M de la cale a la forme générale d'un U, et le plan moyen L' dudit logement est incliné par rapport au plan moyen F' défini par le fond de la rainure 130 d'un angle I' compris entre 60 et 70°. Les angles A et B représentant respectivement l'inclinaison entre une zone de la face interne 134a de la patte 134 et le plan moyen F' défini par le fond de la rainure 130, et l'inclinaison entre une partie du deuxième côté 132a de l'extension 132 et le plan moyen F', sont respectivement sensiblement égaux à 70 et 60°.

La cale étant dépourvue de moyens de fixation au sacrum, les parois du logement 136 sont plus proches l'une de l'autre que dans le premier mode de réalisation, et l'angle existant entre le deuxième côté 132a de l'extension 132 et la face interne 134a de la patte 134 est sensiblement égal à 10°, alors qu'il était plutôt égale à 20° dans le premier mode de réalisation. Ensuite, le fond du logement 136 présente une concavité plus marquée pour recevoir au mieux le bord supérieur de la vertèbre S1 qui est convexe.

En outre, comme représenté figures 6, 7 et 9, une échancrure 150 est ménagée dans l'extension 132, en face de la patte 134, de sorte que lors de la mise en place de la cale, la bosse résiduelle 12 située sur la face postérieure de la vertèbre S1, vient se loger à l'intérieur de l'échancrure 150, ce qui permet d'améliorer la stabilité de la cale 120 sur le sacrum. Le fond 150a de l'échancrure 150 peut être sensiblement parallèle au plan moyen F', ou incliné par rapport à ce plan d'un angle sensiblement égale à 20°, comme représenté figure 9.

La cale 120 présente en outre un trou 152 parallèle au plan F' défini par le fond de la rainure 130, situé en dessous de cette rainure, et traversant le corps 121 de la cale 120. Ce trou débouche dans les première et deuxième faces latérales 126 et 128 du corps de la cale 120, et est prévu pour permettre le passage d'un instrument utilisé pour tenir la cale 120 lors de sa mise en place.

## Revendications

1. Implant intervertébral pour l'articulation lombo-sacrée consistant en une cale (20, 120) apte à être disposée entre la cinquième vertèbre lombaire (L5) et la vertèbre (S1) du sacrum articulée à cette dernière, le corps de ladite cale (21, 121) présentant deux faces d'extrémité supérieure (22, 122) et inférieure (24, 124) opposées, la cale comprenant une rainure (30, 130), orientée suivant le plan médian (M, M') de la cale (20, 120), étant ménagée sur la face d'extrémité supérieure (22, 122) et étant apte à recevoir l'épineuse (10) de ladite vertèbre lombaire (L5), et comprenant un logement longitudinal (36, 136), orienté orthogonalement à ladite rainure (30, 130), étant ménagé sur la face d'extrémité inférieure (24, 124), **caractérisé en ce que** le ledit logement longitudinal (36, 136) est apte à recevoir la partie supérieure (14) de la vertèbre (S1) du sacrum, de sorte que ladite cale (20, 120) soit capable de reposer directement sur cette partie supérieure (14), et **en ce que** le corps de ladite cale présente des première et deuxième faces latérales opposées (26, 126 ; 28, 128) dans lesquelles ladite rainure (30, 130) débouche, et présente à son extrémité inférieure une extension (32, 132) dont un premier côté (32b, 132b) est dans le prolongement de la première face latérale (26, 126), et dont un deuxième côté (32a, 132a), opposé au premier, définit un renfoncement par rapport à la deuxième face latérale (28, 128) du corps de la cale, et **en ce que** la cale (20, 120) comprend une patte (34, 134), de largeur inférieure à la largeur du corps (21, 121) de la cale selon la direction orthogonale au plan médian (M, M') de la cale, raccordée au corps de la cale, et qui s'étend en regard du deuxième côté (32a, 132a) de ladite extension (32, 132), de sorte que la face interne (34a, 134a) de ladite patte (34, 134), orientée face au deuxième côté de l'extension, définit avec ce deuxième côté le contour dudit logement (36, 136).

2. Implant selon la revendication 1, **caractérisé en ce que** la section dudit logement (36, 136), suivant le plan médian (M, M') de la cale, a la forme générale d'un U, et **en ce que** le plan médian (L, L') dudit logement n'est pas orthogonal au plan moyen (F, F') défini par le fond de ladite rainure (30, 130).

3. Implant selon la revendication 2, **caractérisé en ce que** le plan médian (L, L') dudit logement (36, 136) est incliné par rapport au plan moyen (F, F') défini par le fond de ladite rainure (30, 130) d'un angle compris entre 40 et 80°.

4. Implant selon l'une quelconque des revendications précédentes, **caractérisé en ce que** la face interne (34a, 134a) de ladite patte (34, 134) est convexe.

5. Implant selon l'une quelconque des revendications précédentes, **caractérisé en ce qu'**une zone de la face interne (34a, 134a) de la patte est inclinée par rapport au plan moyen (F, F') défini par le fond de ladite rainure (30, 130) d'un angle A compris entre 60 et 80°.

6. Implant selon la revendication 5, **caractérisé en ce que** l'angle A est sensiblement égal à 70°.

7. Implant selon l'une quelconque des revendications précédentes, **caractérisé en ce qu'**une partie du deuxième côté (32a, 132a) de l'extension est inclinée par rapport au plan moyen (F, F') défini par le fond de ladite rainure (30, 130) d'un angle B compris entre 40 et 70°.

8. Implant selon la revendication 7, **caractérisé en ce que** l'angle B est compris entre 50 et 60°.

9. Implant selon l'une quelconque des revendications précédentes, **caractérisé en ce qu'**une échancrure est ménagée dans ladite extension (132), en face de ladite patte (134).

10. Implant selon l'une quelconque des revendications précédentes, **caractérisé en ce que** la cale (20, 120) comprend en outre des premiers moyens de fixation (42, 142) pour fixer le corps (21, 121) de la cale à ladite épineuse (10) de vertèbre lombaire (L5).

11. Implant selon l'une quelconque des revendications précédentes, **caractérisé en ce que** la cale (20) comprend en outre des deuxièmes moyens de fixation (44) pour fixer le corps (21, 121) de la cale à ladite vertèbre (S1) du sacrum.

12. Implant selon la revendication 10 ou 11, **caractérisé en ce que** lesdits moyens de fixation (42, 44, 142) comprennent une sangle (46, 46', 146) et un système d'attache solidaires du corps (21, 121) de ladite cale.

13. Implant selon la revendication 12, **caractérisé en ce qu'**un trou (38) est ménagé dans le corps (21) de ladite cale et débouche dans le fond dudit logement (36), au voisinage de ladite patte (34), et est susceptible de recevoir une portion de sangle (46).

## Claims

1. An intervertebral implant for the lumbo-sacral joint, consisting in a spacer (20, 120) suitable for being placed between the fifth lumbar vertebra (L5) and the sacral vertebra (S1) articulated thereto, the body of said spacer (21, 121) presenting opposite top and bottom end faces (22, 122; 24, 124), the spacer comprising a groove (30, 130) extending along the midplane (M, M') of the spacer (20, 120) being formed in the top end face (22, 122) and being suitable for receiving the spinous process (10) of said lumbar vertebra (L5), and comprising a longitudinal housing (36, 136) extending orthogonally to said groove (30, 130) being formed in the bottom end face (24, 124), **characterized in that** said longitudinal housing (36, 136) is suitable for receiving the top portion (14) of the sacral vertebra (S1) in such a manner that said spacer (20, 120) is capable of resting directly on said top portion (14) and **in that** the body of said spacer presents first and second opposite side faces (26, 126; 28, 128) into which said groove (30, 130) opens out, and presents at its bottom end an extension (32, 132) having a first side (32b, 132b) extending the first side face (26, 126), and a second side (32a, 132a) that is opposite from the first side and that defines a setback relative to the second side face (28, 128) of the body of the spacer, and **in that** the spacer (20, 120) includes a tab (34, 134) of width narrower than the width of the body (21, 121) of the spacer in the direction orthogonal to the midplane (M, M') of the spacer, connected to the body of the spacer, and extending facing the second side (32a, 132a) of said extension (32, 132) in such a manner that the inside face (34a, 134a) of said tab (34, 134) facing the second side of the extension co-operates with said second side to define the outline of said housing (36, 136).

2. An implant according to claim 1, **characterized in that** the section of said housing (36, 136) in the midplane (M, M') of the spacer is generally U-shaped, and **in that** the midplane (L, L') of said housing is not orthogonal to the midplane (F, F') defined by the bottom of said groove (30, 130).

3. An implant according to claim 2, **characterized in that** the midplane (L, L') of said housing (36, 136) is inclined relative to the midplane (F, F') defined by the bottom of said groove (30, 130) by an angle lying in the range 40° to 80°.

4. An implant according to any one of the preceding claims, **characterized in that** the inside face (34a, 134a) of said tab (34, 134) is convex.

5. An implant according to any one of the preceding claims, **characterized in that** a zone of the inside face (34a, 134a) of the tab is inclined relative to the midplane (F, F') defined by the bottom of said groove (30, 130) by an angle A lying in the range 60° to 80°.

6. An implant according to claim 5, **characterized in that** the angle A is substantially equal to 70°.

7. An implant according to any one of the preceding claims, **characterized in that** a portion of the second side (32a, 132a) of the extension is inclined relative to the midplane (F, F') defined by the bottom of said groove (30, 130) at an angle B lying in the range 40° to 70°.

8. An implant according to claim 7, **characterized in that** the angle B lies in the range 50° to 60°.

9. An implant according to any one of the preceding claims, **characterized in that** a notch is formed in said extension (132), the notch facing said tab (134).

10. An implant according to any one of the preceding claims, **characterized in that** the spacer (20, 120) further comprises first fastener means (42, 142) for fastening the body (21, 121) of the spacer to said spinous process (10) of the lumbar vertebra (L5).

11. An implant according to any one of the preceding claims, **characterized in that** the spacer (20) further comprises second fastener means (44) for fastening the body (21, 121) of the spacer to said sacral vertebra (S1).

12. An implant according to claims 10 or 11, **characterized in that** said fastener means (42, 44, 142) comprise a strap (46, 46', 146) and a fastener system secured to the body (21, 121) of said spacer.

13. An implant according to claim 12, **characterized in that** a hole (38) is formed in the body (21) of said spacer and opens out into the bottom of said housing (36) in the vicinity of said tab (34), and is suitable for receiving a portion of a strap (46).

## Patentansprüche

1. Intervertebral-Implantat für das Lumbo-Sakral-Gelenk, das aus einem Keil (20, 120) besteht, der zwischen dem fünften Lendenwirbel (L5) und dem mit diesem gelenkig verbundenen Sakralwirbel (S1) angeordnet werden kann, wobei der Keilkörper (21, 121) zwei einander gegenüberliegende obere (22, 122) und untere Endflächen (24, 124) aufweist, wobei der Keil eine Rille (30, 130) umfaßt, die gemäß der Mittelebene (M, M') des Keils (20, 120) ausgerichtet auf der oberen Endfläche (22, 122) angeordnet ist und den Dornfortsatz (10) des Lendenwirbels (L5) aufnehmen kann, und ein Längslager (36, 136) umfaßt, das orthogonal zur Rille (30, 130) ausgerichtet und auf der unteren Endfläche (24, 124) angeordnet ist, **dadurch gekennzeichnet, daß** das Längslager (36, 136) den oberen Teil (14) des Sakralwirbels (S1) aufnehmen kann, so daß der Keil (20, 120) in der Lage ist, direkt auf diesem oberen Teil (14) aufzuliegen, und **dadurch**, daß der Keilkörper erste und zweite gegenüberliegende Seitenflächen (26, 126; 28, 128) aufweist, in die die Rille (30, 130) mündet, und an seinem unteren Ende eine Erweiterung (32, 132) aufweist, deren erste Seite (32b, 132b) in der Verlängerung der ersten Seitenfläche (26, 126) liegt, und deren zweite Seite (32a, 132a), die der ersten gegenüberliegt, eine Verstärkung bezogen auf die zweite Seitenfläche (28, 128) des Keilkörpers definiert, und **dadurch**, daß der Keil (20, 120) eine Kralle (34, 134) umfaßt, deren Breite kleiner ist als die Breite des Körpers (21, 121) des Keils in der orthogonalen Richtung in der Mittelebene (M, M') des Keils, die mit dem Keilkörper verbunden ist, und die sich gegenüber der zweiten Seite (32a, 132a) der Erweiterung (32, 132) erstreckt, so daß die Innenseite (34a, 134a) der Kralle (34, 134), die gegenüber der zweiten Seite der Erweiterung ausgerichtet ist, mit dieser zweiten Seite die Kontur des Lagers (36, 136) definiert.

2. Implantat nach Anspruch 1, **dadurch gekennzeichnet, daß** der Schnitt des Lagers (36, 136) in der Mittelebene (M, M') des Keils die allgemeine Form eines U hat, und **dadurch**, daß die Mittelebene (L, L') des Lagers nicht orthogonal zur Mittelebene (F, F') liegt, die durch den Boden der Rille (30, 130) definiert ist.

3. Implantat nach Anspruch 2, **dadurch gekennzeichnet, daß** die Mittelebene (L, L') des Lagers (36, 136) bezogen auf die Mittelebene (F, F'), die durch den Boden der Rille (30, 130) definiert ist, in einem Winkel im Bereich zwischen 40 und 80 ° geneigt ist

4. Implantat nach einem der vorhergehenden Ansprüche, **dadurch gekennzeichnet, daß** die Innenfläche (34a, 134a) der Kralle (34, 134) konvex ist.

5. Implantat nach einem der vorhergehenden Ansprüche, **dadurch gekennzeichnet, daß** eine Zone der Innenfläche (34a, 134a) der Kralle bezogen auf die Mittelebene (F, F'), die durch den Boden der Rille (30, 130) definiert ist, in einem Winkel A im Bereich zwischen 60 und 80 ° geneigt ist

6. Implantat nach Anspruch 5, **dadurch gekennzeichnet, daß** der Winkel A im wesentlichen gleich 70 ° ist.

7. Implantat nach einem der vorhergehenden Ansprüche, **dadurch gekennzeichnet, daß** ein Teil der zweiten Seite (32a, 132a) der Erweiterung bezogen auf die Mittelebene (F, F'), die durch den Boden der Rille (30, 130) definiert ist, in einem Winkel B im Bereich zwischen 40 und 70 ° geneigt ist

8. Implantat nach Anspruch 7, **dadurch gekennzeichnet, daß** der Winkel B im Bereich zwischen 50 und 60 ° liegt.

9. Implantat nach einem der vorhergehenden Ansprüche, **dadurch gekennzeichnet, daß** eine Einbuchtung in der Erweiterung (132) gegenüber der Kralle (134) angeordnet ist.

10. Implantat nach einem der vorhergehenden Ansprüche, **dadurch gekennzeichnet, daß** der Keil (20, 120) ferner erste Befestigungsmittel (42, 142) umfaßt, um den Keilkörper (21, 121) an dem Dornfortsatz (10) des Lendenwirbels (L5) zu befestigen.

11. Implantat nach einem der vorhergehenden Ansprüche, **dadurch gekennzeichnet, daß** der Keil (20) ferner zweite Befestigungsmittel (44) umfaßt, um den Keilkörper (21, 121) am Sakralwirbel (S1) zu befestigen.

12. Implantat nach Anspruch 10 oder 11, **dadurch gekennzeichnet, daß** die Befestigungsmittel (42, 44, 142) ein Band (46, 46', 146) und ein Befestigungssystem umfassen, die mit dem Keilkörper (21, 121) fest verbunden sind.

13. Implantat nach Anspruch 12, **dadurch gekennzeichnet, daß** eine Öffnung (38) in dem Keilkörper (21) angeordnet ist und im Boden des Lagers (36) benachbart zur Kralle (34) mündet und in der Lage ist, einen Abschnitt des Bandes (46) aufzunehmen.
